# FASCICULE DE BREVET EUROPEEN

(11) **EP 0 687 273 B1**
(45) Date de publication et mention de la délivrance du brevet: **28.05.1997**
(21) Numéro de dépôt: 94908367.9
(22) Date de dépôt: 23.02.1994
(51) Int. Cl.: C07K 14/81, C07K 1/36, A61K 7/48, A61K 38/55

(54) **INHIBITEUR DE L'ACTIVITE COLLAGENASE ET COMPOSITION COSMETIQUE RENFERMANT UN TEL INHIBITEUR**
INHIBITOR GEGEN KOLLAGENASEAKTIVITÄT UND IHN ENTHALTENDE KOSMETISCHE ZUBEREITUNG
COLLAGENASE ACTIVITY INHIBITOR AND COSMETIC COMPOSITION CONTAINING SAME

(30) Priorité: 05.03.1993 FR 9302782
(43) Date de publication de la demande: 20.12.1995
(73) Titulaire: Groupe Celbert S.A., F-35330 Maure (FR)
(72) Inventeur: BALLESTER, Jean-Michel, F-13005 Marseille (FR); BALLESTER, Maryvonne, F-13005 Marseille (FR); DEGOUL, Vincent, F-13004 Marseille (FR); GIALLO, Jacqueline, F-13015 Marseille (FR); CELBERT, Jean-Marie, F-35380 Plélan-le-Grand (FR)
(74) Mandataire: Vidon, Patrice
(86) Numéro de dépôt international: FR9400200
(87) Numéro de publication internationale: WO9420541

(56) Documents cités:
- FR-A- 2 661 090
- DATABASE WPI Section Ch, Week 9306, Derwent Publications Ltd., London, GB; Class D16, AN 93-049517 & JP,A,5 000 922 (KUROHDA JAPAN KK) 8 Janvier 1993
- PATENT ABSTRACTS OF JAPAN vol. 006, no. 154 (C-119)14 Aoüt 1982 & JP,A,57 072 919 (KISSEI PHARMACEUT CO LTD) 7 Mai 1982
- PATENT ABSTRACTS OF JAPAN vol. 015, no. 181 (C-0830)9 Mai 1991 & JP,A,03 044 331 (LOTTE CO LTD) 26 Février 1991
- PATENT ABSTRACTS OF JAPAN vol. 011, no. 136 (C-419)30 Avril 1987 & JP,A,61 275 224 (SUNSTAR INC) 5 Décembre 1986
- PATENT ABSTRACTS OF JAPAN vol. 011, no. 018 (C-398)17 Janvier 1987 & JP,A,61 192 271 (NITTO SEIFUN KK) 26 Aoüt 1986

## Description

L'invention concerne le domaine de la cosmétologie.

Plus précisément l'invention concerne un inhibiteur montrant des propriétés permettant d'inhiber l'activité de l'enzyme responsable de la dégradation du collagène.

Le collagène est une protéine d'origine muco-polysaccharidique constituant la substance intercellulaire du tissu conjonctif. Le tissu conjonctif, qui se forme chez l'homme à partir du mésenchyme primitif de l'embryon, prend part à la constitution d'organes aussi divers que la peau, les vaisseaux, les os, les tendons, le cartilage, la cornée, etc... Il est constitué essentiellement de cellules, les fibroblastes, et d'une trame fibrillaire formée de trois variétés de fibres/ les fibres réticulées, les fibres élastiques et les fibres de collagène. La protéine collagène formant les fibres de collagène peut exister dans le tissu conjonctif sous trois formes : le collagène neutro-soluble, le collagène acido-soluble et, forme principale, le collagène insoluble, qui a la propriété de se transformer en gélatine sous l'effet de la chaleur et en milieu aqueux. Le tissu conjonctif a un rôle de soutien des organes dans la constitution desquels il entre, rôle assuré essentiellement par les fibres de collagène. La résistance du tissu conjonctif est en effet fonction de la richesse en fibres de collagène et de leur orientation.

Le collagène, étant un des constituants essentiels de la peau, a fait l'objet de nombreuses études qui ont été appliquées, notamment dans le domaine de la cosmétologie, entre autre pour la réalisation de nombreuses compositions à usage cosmétique ayant principalement pour objet d'améliorer l'aspect cutané en favorisant l'hydratation de la couche cornée et en formant un film à la surface de la peau.

Par ailleurs, un axe de la recherche cosmétologique a consisté à rechercher des inhibiteurs de l'enzyme responsable de la dégradation du collagène : la collagénase.

Ces inhibiteurs peuvent présenter des structures chimiques très variées. Il peut s'agir de composés de faibles poids moléculaire (inférieur à 1000 daltons), tels que la cystéine, les sulfonates et notamment le sulfonate d'hydroxyquinoline, la phénantroline, l'EDTA, le trinitrobenzène ou encore l'acétylimidazole. Parmi ces composés de faible poids moléculaire, la cystéine est l'inhibiteur le plus couramment utilisé. Les inhibiteurs de l'activité collagénase peuvent aussi être constitués par des peptides de haut poids moléculaire (supérieur à 1000 daltons) se présentant sous forme de cétones, d'aldéhydes ou encore de thioesters.

De tels inhibiteurs trouvent leur application notamment dans les crèmes anti-rides et plus généralement dans de nombreuses compositions pour le soin de la peau.

Selon leur structure chimique, ces composés sont obtenus soit par synthèse soit par extraction.

L'objet de la présente invention est de fournir un inhibiteur de l'activité enzymatique collagénase extrait d'un produit naturel.

Un autre objectif de l'invention est de proposer un tel inhibiteur pouvant être obtenu selon un procédé d'extraction simple et peu coûteux.

Encore un autre objectif de l'invention est de procurer un tel inhibiteur sous une forme facilement utilisable dans le domaine de la cosmétologie.

Ces différents objectifs sont atteints grâce à l'invention qui concerne un inhibiteur de l'activité enzymatique collagénase caractérisé en ce qu'il est constitué par un extrait protéique de cosses de sarrasin.

Le sarrasin est une plante herbacée annuelle de la famille des polygonacées cultivée pour ses graines riches en amidon qui, une fois réduites en poudre, constituent une farine à usage alimentaire. Une telle farine est notamment employée pour la réalisation des crêpes.

Les fruits du sarrasin se présentent sous la forme d'akènes, constitués par une enveloppe, communément appelée cosse, renfermant une seule graine. Le sarrasin, également appelé "blé noir", est donc une céréale dont l'utilisation pour la fabrication de la farine nécessite une séparation des cosses renfermant les graines, des graines elles-mêmes.

Les cosses de graines de sarrasin ne connaissaient jusqu'ici aucune voie de valorisation et étaient détruites après séparation des graines. La présente invention constitue donc une voie intéressante de valorisation de ce produit secondaire.

Préférentiellement, l'inhibiteur de l'activité collagénase selon l'invention est constitué par un extrait aqueux de cosses de sarrasin.

L'invention concerne aussi un procédé d'extraction d'un tel inhibiteur comprenant les étapes consistant à :
- broyer des cosses de sarrasin dans un solvant aqueux,
- laisser incuber le mélange obtenu à température constante,
- filtrer le mélange obtenu,
- centrifuger le filtrat,
- récupérer le surnageant de centrifugation renfermant ledit extrait protéique,
et caractérisé en ce que ledit solvant est tamponné à un pH basique.

Préférentiellement, le pH de mise en oeuvre de ce procédé est égal à 8.

Egalement préférentiellement, la température à laquelle sont effectuées les différentes étapes du procédé est d'environ 25°C.

Selon une variante du procédé, celui-ci comprend une étape supplémentaire consistant à faire subir une ultrafiltration audit surnageant renfermant ledit extrait protéique.

Selon une autre variante de l'invention, le procédé comprend une étape supplémentaire consistant à faire subir une dialyse audit surnageant renfermant ledit extrait protéique.

Enfin, le procédé comprend préférentiellement une étape finale de lyophilisation.

L'invention concerne également les compositions à usage cosmétique caractérisée en ce qu'elle contiennent au moins un inhibiteur de l'activité collagénase constitué par un extrait protéique de cosses de sarrasin.

L'invention sera plus facilement comprise grâce à la description d'un exemple non-limitatif de réalisation qui va suivre en référence aux dessins dans lesquels :
- la figure 1 représente un graphe traduisant les quantités de protéines obtenues par grammes de cosses de sarrasin en fonction du pH et de la température de mise en oeuvre du procédé.
- la figure 2 représente un graphe traduisant le pourcentage de protéines solubles présentes dans l'extrait obtenu grâce au procédé, en fonction du pH et de la température.
- la figure 3 représente un graphe traduisant la teneur en protéines dans 100 g de produit sec de la fraction soluble, dans différentes conditions de pH et de température.

Un kilogramme de cosses de sarrasin commun (Polygonum fagopyrum) est mélangé avec huit volumes d'un solvant aqueux tamponné présentant un pH égal à 8 (tampon Tris (HCI) 20mM). Le mélange obtenu est ensuite finement broyé et le broyat résultant de cette étape est mis à incuber pendant une heure à 25 °C. Ce broyat est alors filtré sur étamine avant d'être centrifugé. Le surnageant de centrifugation contenant la fraction protéique est récupéré et cette fraction est concentrée par ultrafiltration puis lyophilisation.

A la suite de ce procédé on obtient environ 30 g d'extrait protéique, pour un kilogramme de cosses.

Des essais de mise en oeuvre du procédé ont également été effectués à des pH de 6 et de 9, en remplaçant le tampon précédemment utilisé respectivement par du citrate de sodium 20mM pH 6 et par un tampon Tris (HCI) 20mM pH 9. Chacun de ces essais a été effectué à des températures de 25°C, 37°C et 60°C.

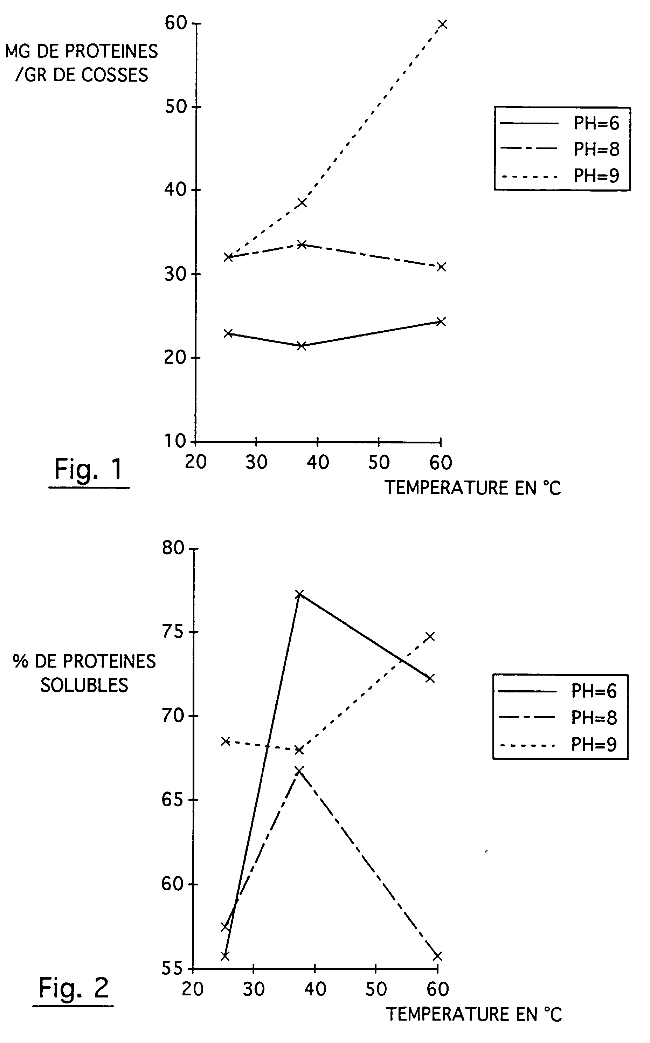

La quantité de protéines obtenue par grammes de cosses est représentée pour chacun de ces essais au tableau I ci-dessous, dont les données sont traduites en graphe à la figure 1. (Cette valeur de 30 g d'extrait protéique est dépendante de la méthode de dosage des protéines. La technique utilisée dans ce cas est la technique au B.C.A. (Bicinchonimic Acid Protein Assay Kit) développée par le laboratoire PIERCE à partir des publications suivantes : LOWRY, O.H. et al. (1951) J. Biol. chem. 193. 265 ; TIKHONOV, V.N. and USTATIN, I.S. (1965) Zh. Anal. Khim. 20, 390; MAZONSKI, T. et al. (1963) Zeszyty. Nauk. Politech. Slask. Chem. 13,63 ; SMITH, P.K. et al. (1985) Anal. Biochem. 150, 76).

**Tableau I**

| pH | Température | mg de protéines/g de cosses |
|---|---|---|
| 6 | 25 | 22 |
| | 37 | 21 |
| | 60 | 23 |
| 8 | 25 | 31 |
| | 37 | 32 |
| | 60 | 31 |
| 9 | 25 | 31 |
| | 37 | 37 |
| | 60 | 59 |

Le tableau II, dont les données sont traduites en graphe à la figure 2 indique le pourcentage de protéines solubles dans la fraction protéique extraite.

**Tableau II**

| pH | Température | % de protéines solubles |
|---|---|---|
| 6 | 25 | 56 |
| | 37 | 78 |
| | 60 | 72 |
| 8 | 25 | 68 |
| | 37 | 67 |
| | 60 | 75 |
| 9 | 25 | 57 |
| | 37 | 66 |
| | 60 | 56 |

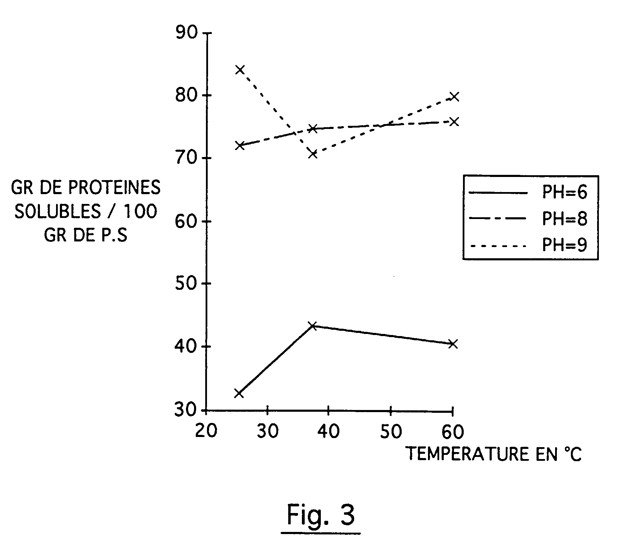

Enfin, le tableau III indique la teneur en protéines dans 100 g de poids sec (P.S.) de la fraction soluble de l'extrait obtenu. Ces données sont reportées à la figure 3.

**Tableau III**

| pH | Température | g de protéines/100g de P.S. |
|---|---|---|
| 6 | 25 | 32 |
| | 37 | 42 |
| | 60 | 41 |
| 8 | 25 | 72 |
| | 37 | 75 |
| | 60 | 76 |
| 9 | 25 | 83 |
| | 37 | 70 |
| | 60 | 79 |

Les données regroupées dans les tableaux I, II et III permettent de constater que la solubilisation minimale des protéines est obtenue à pH = 6, quelle que soit la température à laquelle est mise en oeuvre le procédé. C'est également à ce pH que sont obtenus les plus faibles rendements (environ 20mg de protéines par gramme de cosses). Ce rendement est amélioré de 39 % et porté à environ 30mg de protéines par gramme de cosses lorsque le pH opérationnel est fixé à 8 et ce, également quelle que soit la température opérationnelle. Lorsque le pH utilisé est plus basique (pH = 9), l'extraction des protéines est proportionnelle à la température. Dans les conditions opératoires utilisées, une augmentation de la température opérationnelle de 10°C permet d'accroître la quantité de protéines extraites de 9 g environ. Toutefois, on constate que la solubilité des protéines extraites n'est pas directement proportionnelle à la quantité de protéines obtenues. En effet, au pH le plus acide (pH = 6) et à une température de 37°C, un maximum de protéines est soluble (voir figure 2) alors que l'extraction est minimale (voir figure 1). Un même profil de solubilité est observé à pH = 9. Une solubilité maximale des protéines obtenues à pH 8 et à une température de 60°C est observée. Cependant, il a été également observé que les protéines obtenues à pH 8 et à une température de 60°C ou de 37°C sont relativement instables et ont tendance à précipiter au cours du temps.

L'étude du pourcentage de protéines contenue dans la matière sèche extraite dans la phase aqueuse indique qu'à pH = 6, les protéines ne représente jamais plus de la moitié de la matière extraite, tandis qu'elles en constituent de 72% à 82 % dans le cas de pH basiques.

Les résultats obtenus lors de ces différents essais conduisent à mettre préférentiellement en oeuvre le procédé d'extraction à un pH de 8 et à une température de 25°C environ.

L'activité collagénase a été testée sur l'extrait protéique obtenu après extraction dans ces dernière conditions de pH et de température. Cette activité a été évaluée selon le procédé suivant.

Du collagène à 0,5% a été réparti dans des microcupules d'une plaque de microtitration. Après séchage, 50 µl de l'extrait protéique obtenu puis 20 µl de collagénase à 0,005 % en poids, ont été introduits dans les cupules.

La plaque a été mise à incuber quatre heures à 37°C. Après trois rinçages successifs à l'eau distillée, 100 µl de bleu de Coomassie à 0,25 % ont été ajoutés afin de servir de révélateur à la dégradation du collagène par la collagénase.

Parallèlement, le test a été mené sur une plaque de microtitration en l'absence de l'extrait protéique selon l'invention, cette plaque étant destinée à servir de témoin négatif.

L'inhibition de la dégradation a été évaluée en notant la coloration des cupules après incubation :
- en cas de coloration bleue, le collagène n'est pas dégradé ; l'activité de la collagénase a été inhibée (réaction positive),
- en cas d'absence de coloration, le collagène a été dégradé ; l'activité de la collagénase n'a pas été inhibée (réaction positive).

La mise en oeuvre de ce test avec l'extrait protéique de cosses de sarrasin, a permis de constater que la totalité des cupules avait viré au bleu. Une analyse plus fine, a permis de mettre en évidence que l'extrait protéique de cosses de sarrasin pouvait présenter une activité inhibitrice de la collagénase allant jusqu'à 1600 U par gramme de cosses.

Un tel extrait peut donc être utilisé notamment dans le domaine de la cosmétologie pour réaliser des crèmes ou toute autre préparation destinée à être appliquée sur la peau.

L'objet du présent exemple de réalisation de l'invention n'a pas pour objet de réduire la portée de celle-ci. En particulier, il pourra être envisagé d'extraire la fraction protéique présente dans les cosses de sarrasin selon un autre procédé que celui décrit, sans sortir du cadre de l'invention. Il pourra également être envisagé d'utiliser l'inhibiteur de l'activité collagénase décrit dans d'autres types de préparations cosmétiques que celles indiquées.

## Revendications

1. Inhibiteur de l'activité enzymatique collagénase caractérisé en ce qu'il est constitué par un extrait protéique de cosses de sarrasin.

2. Inhibiteur de l'activité enzymatique collagénase selon la revendication 1 caractérisé en ce que ledit extrait est un extrait aqueux.

3. Procédé d'extraction d'un inhibiteur selon la revendication 2 comprenant les étapes consistant à :
- broyer des cosses de graines de sarrasin dans un solvant aqueux,
- incuber le broyat obtenu à température constante,
- filtrer le mélange obtenu,
- centrifuger le filtrat,
- récupérer le surnageant de centrifugation renfermant ledit extrait protéique,
caractérisé en ce que ledit solvant est tamponné à un pH basique.

4. Procédé d'extraction selon la revendication 3 caractérisé en ce que ledit pH est égal à 8.

5. Procédé d'extraction selon l'une des revendications 3 ou 4 caractérisé en ce que la température à laquelle sont effectuée lesdites étapes est d'environ 25°C.

6. Procédé d'extraction selon l'une des revendications 3 à 5 caractérisé en ce qu'il comprend une étape supplémentaire consistant à faire subir une ultrafiltration audit surnageant renfermant ledit extrait protéique.

7. Procédé d'extraction selon l'une des revendications 3 à 6 caractérisé en ce qu'il comprend une étape supplémentaire consistant à faire subir une dialyse audit surnageant renfermant ledit extrait protéique.

8. Procédé d'extraction selon l'une des revendications 6 ou 7 caractérisé en ce que ledit procédé comprend une étape finale de lyophilisation.

9. Lyophilisat caractérisé en ce qu'il est constitué d'un extrait protéique de cosses de sarrasin.

10. Composition à usage cosmétique caractérisée en ce qu'elle contient au moins un inhibiteur de l'activité collagénase selon l'une des revendications 1 ou 2.

## Patentansprüche

1. Inhibitor der enzymatischen Kollagenaseaktivität,
dadurch gekennzeichnet, daß er aus einem Proteinextrakt von Buchweizenhülsen besteht.

2. Inhibitor der enzymatischen Kollagenaseaktivität gemäß Anspruch 1,
dadurch gekennzeichnet, daß es sich beim Extrakt um einen wäßrigen Extrakt handelt.

3. Extraktionsverfahren für einen Inhibitor gemäß Anspruch 2, der die Schritte umfaßt:
- Zermahlen von Buchweizenhülsen in einem wäßrigen Lösungsmittel,
- Inkubieren des Mahlbreis bei konstanter Temperatur,
- Filtern des erhaltenen Gemisches,
- Zentrifugatieren des Filtrats,
- Aufsammeln des obenauf schwimmenden Zentrifugationsproduktes, welches das Proteinextrakt enthält,
dadurch gekennzeichnet, daß das Lösungsmittel bei einem alkalischen pH-Wert gepuffert ist.

4. Extraktionsverfahren gemäß Anspruch 3,
dadurch gekennzeichnet, daß der pH-Wert 8 beträgt.

5. Extraktionsverfahren gemäß einem der Ansprüche 3 oder 4,
dadurch gekennzeichnet, daß die Ausführungstemperatur der Verfahrensschritte etwa 25 °C beträgt.

6. Extraktionsverfahren gemäß einem der Ansprüche 3 bis 5,
dadurch gekennzeichnet, daß es einen zusätzlichen Schritt umfaßt, wobei das aufschwimmende Produkt, welches den Proteinextrakt enthält, einer Ultrafiltrierung unterworfen wird.

7. Extraktionsverfahren gemäß einem der Ansprüche 3 bis 6,
dadurch gekennzeichnet, daß es einen zusätzlichen Schritt umfaßt, welcher darin besteht, dem obenauf schwimmenden Produkt, welches den Proteinextrakt enthält, einer Dialyse zu unterziehen.

8. Extraktionsverfahren gemäß einem der Ansprüche 6 oder 7,
dadurch gekennzeichnet, daß das Verfahren einen letzten Gefriertrocknungsschritt umfaßt.

9. Gefriertrocknungsprodukt,
dadurch gekennzeichnet, daß es aus einem Buchweizenhülsen-Proteinextrakt besteht.

10. Mischung für kosmetische Zwecke,
dadurch gekennzeichnet, daß es mindestens einen Inhibitor der Kollagenaseaktivität gemäß einem der Ansprüche 1 oder 2 enthält.

## Claims

1. Inhibitor of collegenase enzyme activity characterized in that it consists of a proteinic extract of buckwheat husks.

2. Inhibitor of collegenase enzyme activity according to claim 1, characterized in that the said extract is an aqueous extract.

3. Process for extracting an inhibitor according to claim 2, comprising stages consisting of :
- grinding the buckwheat grain husks with an aqueous solvent,
- incubating the ground product obtained at a constant temperature,
- filtering the mixture obtained,
- centrifuging the filtrate,
- recovering the supernatant after centrifuging containing the said proteinic extract,
characterized in that the said solvent is buffered to a basic pH.

4. Extraction process according to claim 3, characterized in that the said pH is equal to 8.

5. Extraction process according to either of claims 3 or 4, characterized in that the temperature at which said stages are carried out is approximately 25°C.

6. Extraction process according to one of claims 3 to 5, characterized in that it comprises a supplementary stage consisting of subjecting the said supernatant containing the said proteinic extract to ultrafiltration.

7. Extraction process according to one of claims 3 to 6, characterized in that it comprises a supplementary stage consisting of subjecting the said supernatant containing the said proteinic extract to dialysis.

8. Extraction process according to either of claims 6 or 7, characterized in that the said process comprises a final freeze-drying stage.

9. Freeze-dried product, characterized in that it consists of a proteinic extract of buckwheat husks.

10. Composition for cosmetic use, characterized in that it contains at least one inhibitor of collegenase activity according to either of claims 1 or 2.
